(19) Europäisches Patentamt
European Patent Office
Office européen des brevets

(11) Veröffentlichungsnummer: **0 055 442**
**B1**

(12) **EUROPÄISCHE PATENTSCHRIFT**

(45) Veröffentlichungstag der Patentschrift: **08.08.84**

(51) Int. Cl.³: **A 01 N 47/34,** C 07 C 145/04 // C07D251/22, C07D307/52

(21) Anmeldenummer: **81110627.7**

(22) Anmeldetag: **19.12.81**

(54) **N-Sulfenylierte Biurete, Verfahren zu ihrer Herstellung und ihre Verwendung als Fungizide.**

(30) Priorität: **30.12.80 DE 3049439**

(43) Veröffentlichungstag der Anmeldung: **07.07.82 Patentblatt 82/27**

(45) Bekanntmachung des Hinweises auf die Patenterteilung: **08.08.84 Patentblatt 84/32**

(84) Benannte Vertragsstaaten: **AT BE CH DE FR GB IT LI NL SE**

(56) Entgegenhaltungen:
**FR - A - 1 472 975**

**CHEMISCHE BERICHTE, Band 99, Nr. 10, 1966, Seiten 3103-3107, A. HAAS et al.: "Beiträge zur Chemie des Trifluormethylsulfenyl is cyanats und Bis trifluormethylsulfenyl uretidindions"**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., Von-Bodelschwingh-Strasse 42, D-5060 Bergisch-Gladbach 2 (DE)**
Erfinder: **Paul, Volker, Dr., Ahornstrasse 5, D-5650 Solingen 1 (DE)**
Erfinder: **Brandes, Wilhelm, Dr., Eichendorffstrasse 3, D-5653 Leichlingen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 055 442 B1

BUNDESDRUCKEREI BERLIN

**Beschreibung**

Die Erfindung betrifft neue N-sulfenylierte Biurete, Verfahren zu ihrer Herstellung sowie ihrer Verwendung als Fungizide.

Es ist bereits seit langem bekannt, daß N-Trihalogenmethylthio-Verbindungen als Fungizide in der Landwirtschaft verwendet werden können. So werden z. B. das N-(Trichlormethyl-thio)-tetrahydrophthalimid und das N,N-Diemthyl-N'-phenyl-N'-(fluordichlormethylthio)-sulfamid im Obst- und Weinbau zur Bekämpfung von Pilzkrankheiten praktisch eingesetzt (vgl. DE-PS 887 506 (1950) und Angew. Chem. 76, 807 (1964). In tropischen Kulturen, z. B. in Reis, ist ihre Wirkung aber unzureichend.

Weiterhin sind Trifluormethylsulfenyl-carbaminsäureester bzw. -harnstoffderivate mit fungizider Wirkung bekannt. Eine praktische Anwendung dieser Verbindungen kommt wegen der starken pflanzenschädigenden Nebenwirkung nicht in Frage (vgl. Chem. Berichte Bd. 99, Nr. 10, 1966, Seiten 3103—3107).

Es wurden neue N-sulfenylierte Biurete der Formel

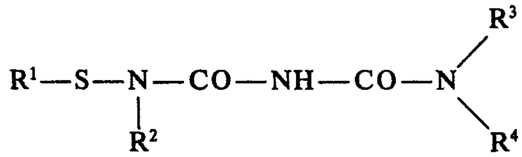

(I)

gefunden, in welcher

$R^1$ für einen Trihalogenmethylrest steht,

$R^2$ für einen gegebenenfalls substituierten aliphatischen, cycloaliphatischen, araliphatischen oder aromatischen Rest steht und

$R^3$ und $R^4$ gleich oder verschieden sein können und für Wasserstoff, einen gegebenenfalls substituierten aliphatischen, cycloaliphatischen, araliphatischen, aromatischen oder heterocyclischen Rest, wobei dieser heterocyclische Rest auch gegebenenfalls über eine Methylengruppe mit dem Stickstoffatom verbunden sein kann, welches die $R^3$- bzw. $R^4$-Substituenten trägt, ferner für die Amino- oder die Hydroxy-Gruppe stehen, oder gemeinsam mit dem verbindlichen Stickstoffatom für einen fünf- oder sechsgliedrigen Heterocyclus stehen können, dessen Ring-Kohlenstoffatome gegebenenfalls durch weitere Heteroatome wie Stickstoff, Sauerstoff oder Schwefel unterbrochen sein können.

Man erhält die N-sulfenylierten Biurete der Formel I, wenn man

a) ein N-(Sulfenamido)-acylisocyanat der Formel

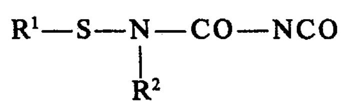

(II)

in welcher

$R^1$ und $R^2$ die oben angegebene Bedeutung haben,

mit einem Amin der Formel

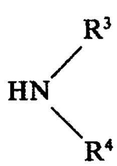

(III)

in welcher

$R^3$ und $R^4$ die oben angegebene Bedeutung haben,

gegebenenfalls in Gegenwart eines Verdünnungsmittels umsetzt, oder

b) einen N-sulfenylierten Allophansäurephenylester der Formel

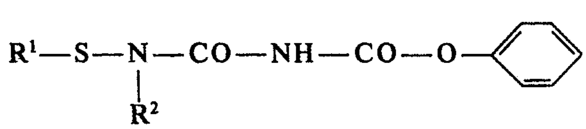

(IV)

in welcher

$R^1$ und $R^2$ die oben angegebene Bedeutung haben,

mit einem Amin der allgemeinen Formel III, gegebenenfalls in Gegenwart eines Verdünnungsmittels, umsetzt.

Die neuen N-sulfenylierten Biurete der Formel I weisen starke fungizide Eigenschaften auf. Überraschenderweise besitzen die erfindungsgemäßen N-sulfenylierten Biurete in verschiedenen Kulturen, wie z. B. in Reis, eine höhere fungizide Wirkung als die bekannten Trihalogenmethylthio-Verbindungen. Sie stellen somit eine Bereicherung der Technik dar.

Von den erfindungsgemäßen Verbindungen der Formel I sind bevorzugt diejenigen, bei denen $R^1$ für die Trichlormethyl- oder Fluordichlormethyl-Gruppe steht, $R^2$ für einen gegebenenfalls durch ein Heteroatom (z. B. Sauerstoff oder Schwefel) unterbrochenen und gegebenenfalls halogenierten aliphatischen Rest mit 1 bis 6 Kohlenstoffatomen, für einen cycloaliphatischen Rest mit 5 bis 8 Kohlenstoffatomen, für einen araliphatischen Rest mit 7 bis 10 Kohlenstoffatomen steht, dessen aromatischer Teil gegebenenfalls durch Halogen, Nitro, Alkyl mit 1 bis 4 Kohlenstoffatomen und/oder Trifluormethyl substituiert sein kann, oder für einen aromatischen Rest mit 6 bis 10 Ring-Kohlenstoffatomen steht, der gegebenenfalls durch Halogen, Nitro, Alkyl mit bis zu 4 Kohlenstoffatomen und/oder Trifluormethyl substituiert sein kann, und $R^3$ und $R^4$ für Wasserstoff, einen gesättigten oder ungesättigten aliphatischen Rest mit 1 bis 6 Kohlenstoffatomen stehen, der gegebenenfalls halogeniert sein und durch Heteroatome wie Sauerstoff, Schwefel oder Stickstoff unterbrochen sein kann, für einen cycloaliphatischen Rest mit 5 bis 7 Ring-Kohlenstoffatomen stehen, der gegebenenfalls durch Alkyl mit 1 bis 4 Kohlenstoffatomen substituiert sein kann, weiterhin für einen araliphatischen Rest mit 7 bis 10 Kohlenstoffatomen stehen, dessen aromatischer Teil gegebenenfalls durch Halogen, Nitro, Alkyl mit 1 bis 4 Kohlenstoffatomen und/oder durch Trifluormethyl substituiert sein kann, sodann noch für einen aromatischen Rest mit 6 bis 10 Ring-Kohlenstoffatomen stehen, der gegebenenfalls substituiert sein kann durch Halogen, Nitro, Alkyl mit 1 bis 4 Kohlenstoffatomen und/oder durch Trifluormethyl, und ferner noch für einen heterocyclischen Rest mit 5 bis 7 Ringatomen stehen, wobei als Heteroatome, Sauerstoff, Schwefel und/oder Stickstoff vorhanden sein können, wobei dieser heterocyclische Rest auch gegebenenfalls über eine $-CH_2$-Gruppe mit dem Stickstoffatom verbunden sein kann, welches die $R^3$- bzw. $R^4$-Substituenten trägt, und schließlich noch für die Amino- oder Hydroxy-Gruppe stehen.

Ganz besonders bevorzugt sind diejenigen Verbindungen der Formel I, in welcher

$R^1$ für eine Trichlormethyl- oder Fluordichlormethyl-Gruppe steht,
$R^2$ für eine gegebenenfalls durch Methoxy oder Ethoxy substituierte Alkyl-Gruppe mit 1 bis 4 Kohlenstoffatomen, für Cyclohexyl oder für Phenyl steht,
$R^3$ für Wasserstoff, eine Alkyl-Gruppe mit bis zu 6 oder eine Alkenyl-Gruppe mit bis zu 4 Kohlenstoffatomen steht, für eine Halogenalkyl-Gruppe mit bis zu 4 Kohlenstoff- und bis zu 5 Halogenatomen steht, ferner für Phenyl, Halogenphenyl, Cyclohexyl, eine Furfuryl- oder Triazin-Gruppe steht, wobei letztere noch durch Methyl-Gruppen substituiert sein kann, und schließlich noch für eine Amino-Gruppe steht, und
$R^4$ für Wasserstoff oder Alkyl mit bis zu 6 Kohlenstoffatomen steht.

Verwendet man zum Beispiel zur Herstellung der erfindungsgemäßen Verbindungen der Formel I gemäß Verfahrensvariante a) N-(Fluordichlormethylsulfenyl) -N-methyl-carbamoylisocyanat oder gemäß Variante b) N-(Fluordichlormethylsulfenyl)-N-methyl-allophansäurephenylester und in beiden Fällen Ethylamin als Ausgangskomponente, so kann der Reaktionsverlauf durch das nachfolgende Reaktionsschema wiedergegeben werden:

a) 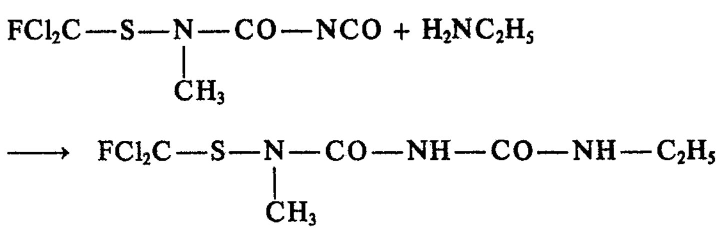

b) 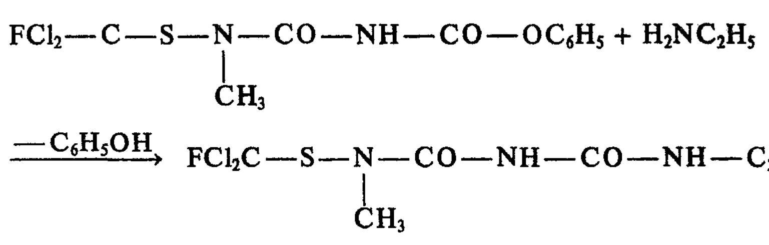

Die als Ausgangsstoffe für die Herstellungsverfahrens-Variante a) benötigten N-(Sulfenamido)-acylisocyanate sind durch die allgemeine Formel II definiert.

In dieser Formel haben $R^1$ und $R^2$ vorzugsweise die Bedeutungen, die bei Besprechung der erfindungsgemäßen Verbindungen der Formel I für diese Substituenten bereits vorzugsweise genannt worden sind. Die Verbindungen der Formel II sind noch nicht bekannt; sie sind Gegenstand einer getrennten Anmeldung (vergleiche DE-OS 3 049 448).

Man erhält sie, wenn man Trihalogenmethan-sulfenamide der Formel

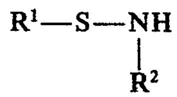 (V)

in welcher $R^1$ und $R^2$ die oben angegebene Bedeutung besitzen, mit Chlorcarbonylisocyanat der Formel

$$Cl—CO—NCO \quad (VI)$$

gegebenenfalls in Gegenwart eines Verdünnungsmittels im Temperaturbereich zwischen 0 und 150° C umsetzt.

Für das Herstellungsverfahren a) geeignete N-(Sulfenamido)-acylisocyanate der Formel II sind die Acylisocyanate des N-(Trichlormethylsulfenyl)-, N-(Fluordichlormethylsulfenyl)- und N-(Trifluormethylsulfenyl)-methylamins, -isopropylamins, -2-methoxiethylamins, -tert.-butylamins, -cyclopentylamins, -cyclohexylamins, -benzylamins, -4-chlorbenzylamins, -phenethylamins, -anilins, -3-trifluor-methylanilins, -3, 4-dichloranilins, -4-anisidins und -3-toluidins.

Die Vorprodukte der Formel V sind bekannt (vgl. FR-PS 13 39 765 bzw. Chem. Abstr. 60, 5519 (1964)); sie werden erhalten, wenn man ein Trihalogenmethansulfenchlorid mit einem primären Amin beispielsweise in Toluol als Lösungsmittel im Temperaturbereich zwischen +20 und 30° C zur Umsetzung bringt. Das Vorprodukt der Formel VI ist ebenfalls bekannt (Angew. Chem. 89, 789 (1977)), es wird durch partielle Verseifung des Additionsproduktes aus Phosgen und Chlorcyan erhalten.

Die als Ausgangsprodukte für die Herstellungsverfahrensvariante b) benötigten N-sulfenylierten Allophansäurephenylester sind durch die obige allgemeine Formel IV definiert. In dieser Formel haben $R^1$ und $R^2$ die weiter oben angegebenen vorzugsweisen Bedeutungen. Die Verbindungen werden erhalten, wenn man an die weiter oben angegebenen Trihalogenmethan-sulfenamide der Formel V Phenoxicarbonylisocyanat der Formel

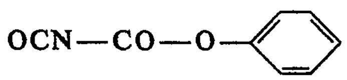 (VII)

addiert (vgl. auch die Herstellungsbeispiele).

Die für beide Verfahrenswege a) und b) benötigten Amine sind durch die allgemeine Formel III definiert. In dieser Formel haben die Substituenten $R^3$ und $R^4$ vorzugsweise die Bedeutungen, die bei Besprechung der Verbindungen der Formel I bereits für diese Substituenten vorzugsweise genannt worden sind. Die Verbindungen sind allgemein bekannt. Zu nennen sind z. B. Ammoniak, Methyl-, Dimethyl-, Ethyl-, Isopropyl-, Allyl-, Trifluorethyl-, Methoxyethyl-, tert.-Butyl-, Cyclopentyl-, 2-Methylcyclohexyl-, 4-Chloranilin, 4-Fluoranilin, 3-Anisidin, 1-Naphthylamin, 2-Aminopyridin, 2-Aminothiophen und 2-Aminofuran.

Bei der Durchführung der Herstellung nach Verfahrensvariante a) kommen als Verdünnungsmittel alle inerten organischen Lösungsmittel in Frage. Hierzu gehören vorzugsweise Kohlenwasserstoffe, wie Benzin, Benzol oder Toluol, Ether, wie Diethylether, und Ketone, wie Aceton und Methylethylketon.

Die Reaktionstemperaturen können beim Verfahren a) in einem größeren Bereich variiert werden. Im allgemeinen arbeitet man zwischen etwa −10 und +100° C, vorzugsweise zwischen +20 und 50° C.

Arbeitet man nach dem Herstellungsverfahren b), so löst man das N-sulfenylierte Allophanat der Formel IV in einem für das Endprodukt schwerlöslichen Lösungsmittel, vorzugsweise in niederen aliphatischen Alkoholen oder in deren wäßrigen Mischungen. Hierdurch erfolgt eine leichte Abtrennung von dem bei der Reaktion entstehenden Phenol. Man führt diese Reaktion bei −10 bis +100° C, vorzugsweise bei +10 bis 50° C durch.

Bei der Durchführung der Umsetzung nach Verfahrensvariante a) setzt man auf 1 Mol N-(Sulfenamido)-acylisocyanat der Formel II etwa 1 Mol des Amins der Formel III ein. Die Isolierung der erfindungsgemäßen Verbindungen erfolgt im allgemeinen durch Kristallisation.

Bei der Durchführung der Umsetzung nach Verfahrensvariante b) legt man den N-sulfenylierten Allophansäurephenylester der Formel IV, in einem niederen Alkohol gelöst, vor, und fügt mindestens die äquimolare Menge des Amins (III), gegebenenfalls in wäßriger oder alkoholischer Lösung, hinzu. Das Endprodukt der Formel I kristallisiert aus und wird in üblicher Weise abgetrennt.

Die erfindungsgemäßen Wirkstoffe weisen eine starke mikrobizide Wirkung auf und können zur

Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Pyricularia oryzae, dem Erreger der Brusone-Krankheit und Pellicularia sasakii, dem Erreger der Blattscheidendürre an Reis, eingesetzt werden. Gute Erfolge werden auch gegen Apfelschorf (Venturia inaequalis) und Grauschimmel (Botrytis cinerea) erzielt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalino, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

(Verfahrensvariante a)

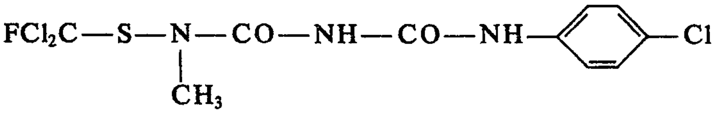

8,2 g (0,035 Mol) N-(Fluordichlormethansulfenyl)-N-(methyl)-amidocarbonylisocyanat werden in 60 ml Aceton gelöst und bei Raumtemperatur mit der Lösung von 4,5 g (0,035 Mol) 4-Chloranilin in 20 ml Aceton umgesetzt. Hierbei steigt die Temperatur bis 34° C an. Auf Zusatz von Wasser erhält man 7 g 1-Fluordichlormethylthio-1-methyl-5-(4-chlorphenyl)-biuret vom Fp. 148° C, das sind 55% der Theorie.

### Beispiel 2

(Verfahrensvariante b)

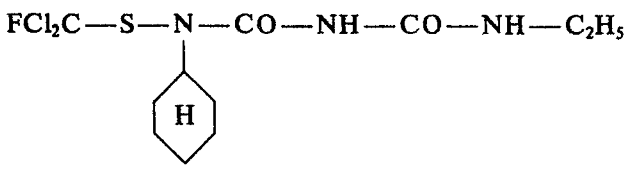

39,5 g (0,1 Mol) N-(Fluordichlormethylsulfenyl)-N-(cyclo-hexyl)-allophansäurephenylester werden in 50 ml Methanol gelöst und unter Eiswasserkühlung bei 10 bis 20° C mit 15 ml einer 50%igen wäßrigen Ethylamin-Lösung versetzt. Hierbei kristallisiert das Reaktionsprodukt aus. Man saugt das Produkt ab, löst in 30 ml heißem Methanol und gibt bei 50°C 15 ml Wasser zu. Das in der Kälte anfallende Reaktionsprodukt wird abgesaugt und getrocknet. Man erhält 26 g 1-Fluordichlormethylthio-1-cyclo-hexyl-5-ethyl-biuret vom Fp. 104—105° C, das sind 75% der Theorie.

In analoger Weise können die folgenden Verbindungen der allgemeinen Formel

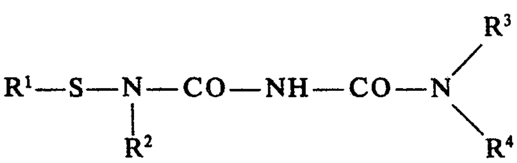 (I)

erhalten werden:

| Beispiel Nr. | $R^1$ | $R^2$ | $R^3$ | $R^4$ | Fp. (°C) |
|---|---|---|---|---|---|
| 3 | $CCl_3$ | $C_4H_9$-n | $CH_3$ | $CH_3$ | 78 |
| 4 | $CCl_3$ | $C_4H_9$-n | $C_2H_5$ | H | 48 |
| 5 | $CCl_3$ | $C_4H_9$-n | 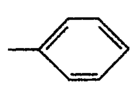 | H | 83 |
| 6 | $CCl_3$ | 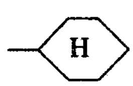 | $CH_3$ | H | 110 |
| 7 | $CCl_3$ | 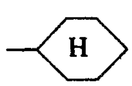 | $C_2H_5$ | H | 108 |
| 8 | $CCl_3$ | 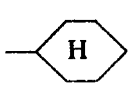 | $C_3H_7$-i | H | 86 |

Fortsetzung

| Beispiel Nr. | $R^1$ | $R^2$ | $R^3$ | $R^4$ | Fp. (°C) |
|---|---|---|---|---|---|
| 9 | $CCl_3$ | 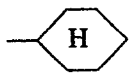 | 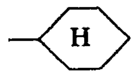 | H | 125 |
| 10 | $CCl_2F$ | $CH_3$ | $CH_3$ | H | 102 |
| 11 | $CCl_2F$ | $CH_3$ | $C_2H_5$ | H | 65 |
| 12 | $CCl_2F$ | $CH_3$ | $C_3H_7$-n | H | 48 |
| 13 | $CCl_2F$ | $CH_3$ | $CH_2{-}CH{=}CH_2$ | H | 57 |
| 14 | $CCl_2F$ | $CH_3$ | $C_3H_7$-i | H | 89 |
| 15 | $CCl_2F$ | $CH_3$ | $C_4H_9$-t | H | 83 |
| 16 | $CCl_2F$ | $CH_3$ | 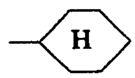 | H | 117 |
| 17 | $CCl_2F$ | $CH_3$ | 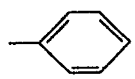 | H | 138 |
| 18 | $CCl_2F$ | $C_3H_7$-i | $C_2H_5$ | H | 82 |
| 19 | $CCl_2F$ | $C_3H_7$-i | 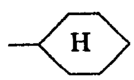 | H | 89 |
| 20 | $CCl_2F$ | $C_4H_9$-t | $CH_3$ | H | 87 |
| 21 | $CCl_2F$ | $C_4H_9$-t | $C_2H_5$ | H | 86 |
| 22 | $CCl_2F$ | $C_4H_9$-t | 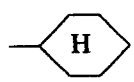 | H | 111 |
| 23 | $CCl_2F$ | 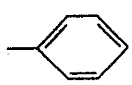 | $C_2H_5$ | H | 109 |
| 24 | $CCl_2F$ | 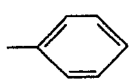 | 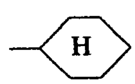 | H | 109 |
| 25 | $CCl_2F$ | 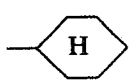 | $CH_3$ | $CH_3$ | 117 |
| 26 | $CCl_2F$ | 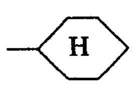 | H | H | 153 |
| 27 | $CCl_2F$ | 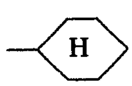 | $C_3H_7$-i | H | 80 |
| 28 | $CCl_2F$ | 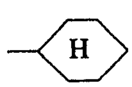 | 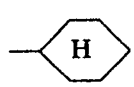 | H | 131 |
| 29 | $CCl_2F$ | 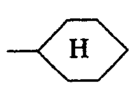 | $C_4H_9$-t | H | 108 |
| 30 | $CCl_2$-F | 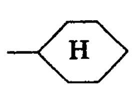 | $CH_2{-}C(CH_3)_3$ | H | 134 |

Fortsetzung

| Beispiel Nr. | $R^1$ | $R^2$ | $R^3$ | $R^4$ | Fp. (°C) |
|---|---|---|---|---|---|
| 31 | $CCl_2$-F | 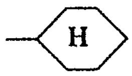 | $CH_2$— 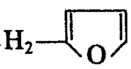 | H | 86 |
| 32 | $CCl_2$-F | 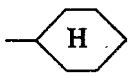 | 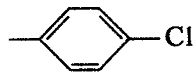 | H | 155 |
| 33 | $CCl_2F$ | $CH_3$ | 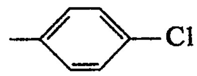 | H | 148 |
| 34 | $CCl_2F$ | $CH_2-CH_2-OCH_3$ | $C_3H_7$-i | H | |
| 35 | $CCl_2F$ | 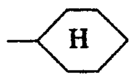 | $CH_2—CF_3$ | $CH_3$ | 84–85 |
| 36 | $CCl_2F$ | 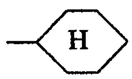 | $CH_2—CF_3$ | H | $n_D^{20}$: 1,5001 |
| 37 | $CCl_2F$ | 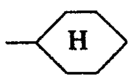 | $CH_2—CH_2—CF_3$ | H | 105 |
| 38 | $CCl_2F$ | 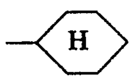 | $CH(CH_3)—CF_3$ | H | 140 |
| 39 | $CCl_2F$ | 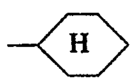 | $CH_2—CH_2—CFCl_2$ | H | $n_D^{20}$: 1,5177 |
| 40 | $CCl_2F$ | 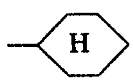 | $CH_2—CF_3$ | $C_2H_5$ | $n_D^{20}$: 1,4918 |
| 41 | $CCl_2F$ | $C_4H_9$-t | $NH_2$ | H | 125–126 |
| 42 | $CCl_2F$ | $C_3H_7$-i | $C_4H_9$-t | H | $n_D^{20}$: 1,5012 |
| 43 | $CCl_2F$ | $C_3H_7$-i | $NH_2$ | H | 130 |
| 44 | $CCl_2F$ | $C_4H_9$-n | 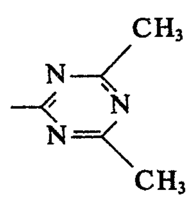 | H | 126–129 |
| 45 | $CCl_2F$ | 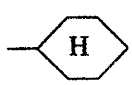 | $NH_2$ | H | 119–121 |
| 46 | $CCl_2F$ | 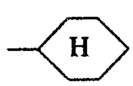 | $CH_3$ | H | 107–109 |
| 47 | $CCl_2F$ | 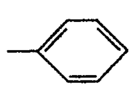 | 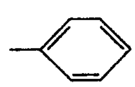 | H | 139–141 |

Herstellung der Vorprodukte

Beispiel V1

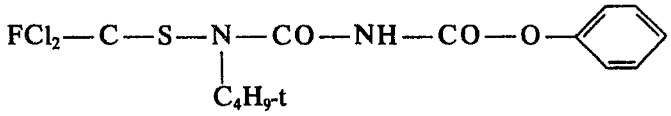

Zu 48,8 g (0,3 Mol) Phenoxicarbonylisocyanat, gelöst in 150 ml trockenem Toluol, tropft man bei Raumtemperatur 62 g (0,3 Mol) des aus Fluordichlormethansulfenylchlorid und tert.-Butylamin hergestellten (Fluordichlormethansulfenyl)N-tert.-butylamids ($Kp_{13}$/60—65° C), gelöst in 30 ml Toluol. Hierbei steigt die Temperatur bis 44° C an. In der Kälte kristallisieren 39 g der obigen Verbindung vom Fp. 101—102° C aus. Aus der Mutterlauge können durch Zusatz von Petrolether weitere 34 g des Produktes gewonnen werden.

In ähnlicher Weise erhält man die folgenden Vorprodukte.

V 2 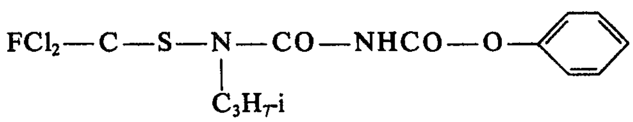 $n_D^{20}$: 1,5379

V 3 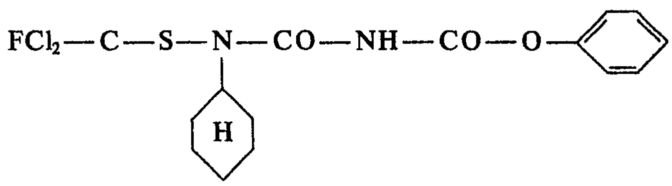 $n_D^{20}$: 1,5389

V 4 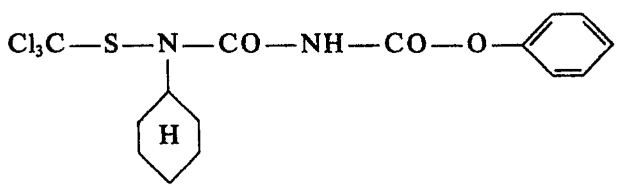 $n_D^{20}$: 1,5570

Anwendungsbeispiele

In den nachfolgenden Anwendungsbeispielen wurde zum Vergleich mit dem Stande der Technik die folgende Verbindung herangezogen:

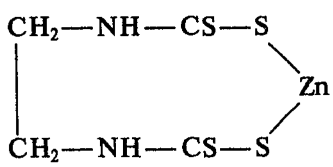

Beispiel A

Pyricularia-Test (Reis)/protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25° C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z. B. die Verbindungen gemäß folgender Herstellungsbeispiele: 2 und 29.

## Beispiel B

### Pellicularia-Test (Reis)

| | |
|---|---|
| Lösungsmittel: | 12,5 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3 bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25° C und 100% relativer Luftfeuchtigkeit aufgestellt.

5—8 Tage nach der Inokulation erfolgt die Auswertung des Krankheitsbefalles.

Eine deutliche Überlegenheit in der Wirksamkeit gegenüber dem Stand der Technik zeigen bei diesem Test z. B. die Verbindungen gemäß folgender Herstellungsbeispiele: 2 und 29.

## Beispiel C

### Botrytis-Test (Bohnen)/protektiv

| | |
|---|---|
| Lösungsmittel: | 4,7 Gewichtsteile Aceton |
| Dispergiermittel: | 0,3 Gewichtsteile Alkyl-aryl-polyglykolether |
| Wasser: | 95 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2 Blattstadium bis zur Tropfnässe. Nach 24 Stunden werden auf jedes Blatt 2 kleine mit Botrytis cinerea bewachsene Agarstückchen aufgelegt. Die inokulierten Pflanzen werden in einer abgedunkelten, feuchten Kammer bei 20° C aufgestellt. 3 Tage nach der Inokulation wird die Größe der Befallsflecken auf den Blättern bonitiert.

Die erhaltenen Boniturwerte werden auf Prozent Befall umgerechnet. 0% bedeutet keinen Befall, 100% bedeutet, daß der Befallsfleck vollständig ausgebildet ist.

Eine deutliche Überlegenheit in der Wirksamkeit gegenüber dem Stand der Technik zeigen bei diesem Test z. B. die Verbindungen gemäß folgender Herstellungsbeispiele: 45, 46, 17, 16 und 47.

**Patentansprüche**

1. N-Sulfenylierte Biurete der Formel

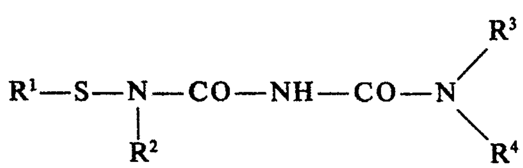

(I)

in welcher

$R^1$ für einen Trihalogenmethylrest steht,

$R^2$ für einen gegebenenfalls substituierten aliphatischen, cycloaliphatischen, araliphatischen oder aromatischen Rest steht und

$R^3$ und $R^4$ gleich oder verschieden sein können und für Wasserstoff, einen gegebenenfalls substituierten aliphatischen, cycloaliphatischen, araliphatischen, aromatischen oder heterocyclischen Rest, wobei dieser heterocyclische Rest auch gegebenenfalls über eine Methylengruppe mit dem Stickstoffatom verbunden sein kann, welches die $R^3$- bzw. $R^4$-Substituenten trägt, ferner für die Amino- oder die Hydroxy-Gruppe stehen, oder gemeinsam mit dem verbindenden Stickstoffatom für einen fünf- oder sechsgliedrigen Heterocyclus stehen können, dessen Ring-Kohlenstoffatome gegebenenfalls durch weitere Heteroatome wie Stickstoff, Sauerstoff oder Schwefel unterbrochen sein können.

2. N-Sulfenylierte Biurete der Formel I in Anspruch 1, in welcher

$R^1$ für eine Trichlormethyl- oder Fluordichlormethyl-Gruppe steht,

$R^2$ für eine gegebenenfalls durch Methoxy oder Ethoxy substituierte Alkyl-Gruppe mit 1 bis 4 Kohlenstoffatomen, für Cyclohexyl oder für Phenyl steht,

$R^3$ für Wasserstoff, eine Alkyl-Gruppe mit bis zu 6 oder eine Alkenyl-Gruppe bis zu 4 Kohlenstoffatomen steht, für eine Halogenalkyl-Gruppe mit bis zu 4 Kohlenstoff- und bis zu 5 Halogenatomen steht, ferner für Phenyl, Halogenphenyl, Cyclohexyl, eine Furfuryl- oder Triazin-Gruppe steht, wobei letztere noch durch Methyl-Gruppen substituiert sein kann, und schließlich noch für eine Amino-Gruppe steht, und

$R^4$ für Wasserstoff oder Alkyl mit bis zu 6 Kohlenstoffatomen steht.

3. Verfahren zur Herstellung von N-sulfenylierten Biureten der Formel

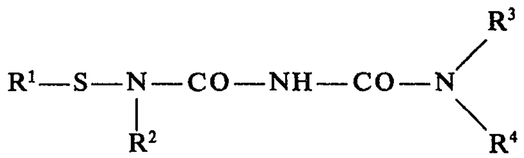 (I)

in welcher

$R^1$ für einen Trihalogenmethylrest steht,

$R^2$ für einen gegebenenfalls substituierten aliphatischen, cycloaliphatischen, araliphatischen oder aromatischen Rest steht und

$R^3$ und $R^4$ gleich oder verschieden sein können und für Wasserstoff, einen gegebenenfalls substituierten aliphatischen, cycloaliphatischen, araliphatischen, aromatischen oder heterocyclischen Rest, wobei dieser heterocyclische Rest auch gegebenenfalls über eine Methylengruppe mit dem Stickstoffatom verbunden sein kann, welches die $R^3$- bzw. $R^4$-Substituenten trägt, ferner für die Amino- oder die Hydroxy-Gruppe stehen oder gemeinsam mit dem verbindenden Stickstoffatom für einen fünf- oder sechsgliedrigen Heterocyclus stehen können, dessen Ring-Kohlenstoffatome gegebenenfalls durch weitere Heteroatome wie Stickstoff, Sauerstoff oder Schwefel unterbrochen sein können;

dadurch gekennzeichnet, daß man

a) ein N-(Sulfenamido)-acylisocyanat der Formel

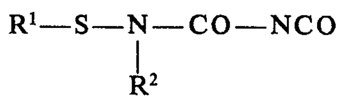 (II)

in welcher
$R^1$ und $R^2$ die oben angegebene Bedeutung haben,

mit einem Amin der Formel

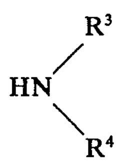 (III)

in welcher
$R^3$ und $R^4$ die oben angegebene Bedeutung haben,

gegebenenfalls in Gegenwart eines Verdünnungsmittels umsetzt, oder

b) einen N-sulfenylierten Allophansäurephenylester der Formel

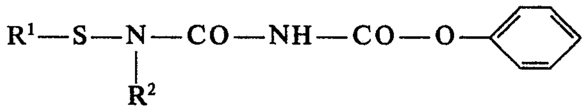 (IV)

in welcher
$R^1$ und $R^2$ die oben angegebene Bedeutung haben,

mit einem Amin der allgemeinen Formel III, gegebenenfalls in Gegenwart eines Verdünnungsmittels, umsetzt.

4. Fungizide Mittel, gekennzeichnet durch einen Gehalt an mindestens einem N-sulfenylierten Biuret der Formel I in Anspruch 1 und 3.

5. Verfahren zur Bekämpfung von Pilzen, dadurch gekennzeichnet, daß man N-sulfenylierte Biurete der Formel I in Ansprüchen 1 und 3 auf Pilze oder ihren Lebensraum einwirken läßt.

6. Verwendung von N-sulfenylierten Biureten der Formel I in Ansprüchen 1 und 3 zur Bekämpfung von Pilzen.

7. Verfahren zur Herstellung von fungiziden Mitteln, dadurch gekennzeichnet, daß man N-sulfenylierte Biurete der Formel I in Ansprüchen 1 und 3 mit Streckmitteln und/oder oberflächenaktiven Mitteln vermischt.

**Claims**

1. N-Sulphenylated biurets of the formula

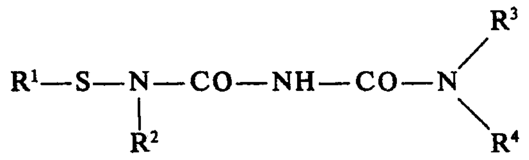

(I)

in which

$R^1$ represents a trihalogenomethyl radical,

$R^2$ represents an optionally substituted aliphatic, cycloaliphatic, araliphatic or aromatic radical and

$R^3$ and $R^4$

may be identical or different and represent hydrogen, an optionally substituted aliphatic, cycloaliphatic, araliphatic, aromatic or heterocyclic radical, it also being possible for this heterocyclic radical to be optionally bonded via a methylene group to the nitrogen atom carrying the $R^3$ and $R^4$ substituents, or the amino or hydroxyl group, or together with the connecting nitrogen atom can represent a five-membered or six-membered heterocyclic ring, the ring carbon atoms of which can optionally be interrupted by further hetero-atoms, such as nitrogen, oxygen or sulphur.

2. N-Sulphenylated biurets of the formula I in claim 1,

in which

$R^1$ represents a trichloromethyl or fluorodichloromethyl group,

$R^2$ represents an alkyl group which has 1 to 4 carbon atoms and is optionally substituted by methoxy or ethoxy, or cyclohexyl or phenyl,

$R^3$ represents hydrogen, an alkyl group with up to 6 carbon atoms or an alkenyl group up to 4 carbon atoms, a halogenoalkyl group with up to 4 carbon atoms and up to 5 halogen atoms, or phenyl, halogenophenyl, cyclohexyl, a furfuryl group or a triazine group, it being possible for the latter to be further substituted by methyl groups, or, finally, an amino group, and

$R^4$ represents hydrogen or alkyl with up to 6 carbon atoms.

3. Process for the preparation of N-sulphenylated biurets of the formula

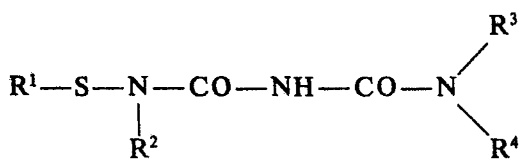

(I)

in which

$R^1$ represents a trihalogenomethyl radical,

$R^2$ represents an optionally substituted aliphatic, cycloaliphatic, araliphatic or aromatic radical and

$R^3$ and $R^4$

may be identical or different and represent hydrogen, an optionally substituted aliphatic, cycloaliphatic, araliphatic, aromatic or heterocyclic radical, it also being possible for this heterocyclic radical to be optionally bonded via a methylene group to the nitrogen atom carrying the $R^3$ and $R^4$ substituents, or the amino or hydroxyl group, or together with the connecting nitrogen atom can represent a five-membered or six-membered heterocyclic ring, the ring carbon atoms of which can optionally be interrupted by further hetero-atoms, such as nitrogen, oxygen or sulphur;

characterized in that

a) a N-(sulphenamido)-acyl isocyanate of the formula

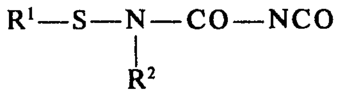

(II)

in which
$R^1$ and $R^2$ have the abovementioned meaning,

are reacted with an amine of the formula

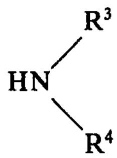

(III)

in which
$R^3$ and $R^4$ have the abovementioned meaning,

if appropriate in the presence of a diluent, or

b) a N-sulphenylated allophanic acid phenyl ester of the formula

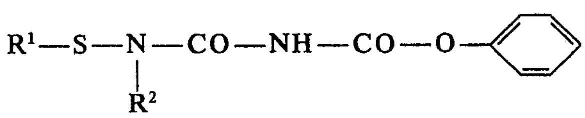

(IV)

in which
$R^1$ and $R^2$ have the abovementioned meaning,

is reacted with an amine of the general formula III, if appropriate in the presence of a diluent.

4. Fungicidal agents, characterized in that they contain at least one N-sulphenylated biuret of the formula I in claim 1 and 3.

5. Process for combating fungi, characterized in that N-sulphenylated biurets of the formula I in claims 1 and 3 are allowed to act on fungi or their environment.

6. Use of N-sulphenylated biurets of the formula I in claims 1 and 3 for combating fungi.

7. Process for the preparation of fungicidal agents, characterized in that n-sulphenylated biurets of the formula I in claims 1 and 3 are mixed with extenders and/or surface-active agents.

## Revendications

1. Biurets N-sulfénylés, de formule

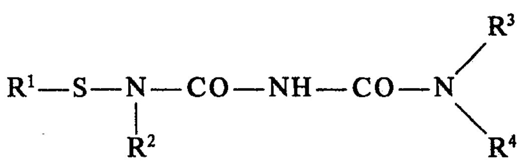

(I)

dans laquelle

$R^1$ représente un radical trihalogénométhyle,

$R^2$ représente un radical aliphatique, cycloaliphatique, araliphatique ou aromatique éventuellement substitués, et

$R^3$ et $R^4$
peuvent être identiques ou différents et ils représentent l'hydrogène, un radical aliphatique, cycloaliphatique, araliphatique, aromatique ou hétérocyclique éventuellement substitués (ce radical hétérocyclique pouvant aussi être éventuellement relié par un groupe méthylène à l'atome d'azote qui porte les substituants $R^3$ ou $R^4$), ainsi que le groupe amino ou hydroxyle, ou bien, pris avec l'atome d'azote qui les relie, ils peuvent représenter un hétérocycle pentagonal ou hexagonal, dont les atomes de carbone de cycle peuvent être interrompus par d'autres hétéroatomes comme l'azote, l'oxygène ou le soufre.

2. Biurets N-sulfénylés de formule I de la revendication 1,

dans lesquels

$R^1$ représente un groupe trichlorométhyle ou fluorodichlorométhyle,

$R^2$ représente un groupe alkyle ayant 1 à 4 atomes de carbone éventuellement substitués par un groupe méthoxy ou éthoxy ou représente un groupe un groupe cyclohexyle ou phényle,

$R^3$ représente l'hydrogène, un groupe alkyle ayant jusqu'à 6 atomes de carbone ou un groupe alcényle ayant jusqu'à 4 atomes de carbone, un groupe halogénoalkyle ayant jusqu'à 4 atomes de carbone et jusqu'à 5 atomes d'halogène, ainsi qu'un groupe phényle, halogénophényle, cyclohexyle, furfuryle ou triazine (ce dernier pouvant encore être substitué par des groupes méthyles) et enfin, encore, un groupe amino, et

$R^4$ représente l'hydrogène ou un groupe alkyle ayant jusqu'à 6 atomes de carbone.

3. Procédé de préparation de biurets N-sulfénylés de formule

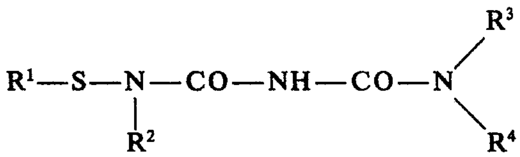

(I)

dans laquelle

$R^1$ représente un radical trihalogénométhyle,

$R^2$ représente un radical aliphatique, cycloaliphatique, araliphatique ou aromatique éventuellement substitués, et

$R^3$ et $R^4$

peuvent être identiques ou différents et ils représentent de l'hydrogène, un radical aliphatique, cycloaliphatique, aromatique ou hétérocyclique, éventuellement substitués (ce radical hétérocyclique pouvant éventuellement aussi être relié par un groupe méthylène à l'atome d'azote qui porte les substituants $R^3$ ou $R^4$), ainsi que le groupe amino ou le groupe hydroxyle ou bien, pris avec l'atome d'azote qui les relie, ils peuvent représenter un hétérocycle pentagonal à hexagonal, dont les atomes de carbone de cycle peuvent éventuellement être interrompus par d'autres hétéroatomes comme l'azote, l'oxygène ou le soufre, procédé caractérisé en ce que

a) on fait réagir un isocyanate de N-(sulfénamido)-acyle de formule

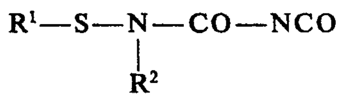

(II)

dans laquelle

$R^1$ et $R^2$ ont le sens indiqué ci-dessus, avec une amine de formule

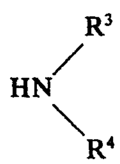

(III)

dans laquelle

$R^3$ et $R^4$ ont le sens indiqué ci-dessus, éventuellement en présence d'un diluant, ou

b) on fait réagir un allophanate de phényle N-sulfénylé de formule

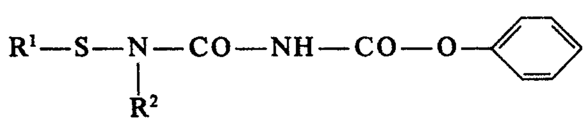

(IV)

dans laquelle

$R^1$ et $R^2$ ont le sens indiqué ci-dessus,

avec une amine de formule générale III, éventuellement en présence d'un diluant.

4. Produit fongicide, caractérisé en ce qu'il contient au moins un biuret N-sulfénylé de formule I selon la revendication 1 et 3.

5. Procédé pour lutter contre les champignons, caractérisé en ce qu'on fait agir des biurets N-sulfénylés de formule I des revendications 1 et 3 sur les champignons ou sur leur biotope.

6. Utilisation de biurets N-sulfénylés de formule I des revendications 1 et 3 pour la lutte contre les champignons.

7. Procédé de préparation de produits fongicides, caractérisé en ce qu'on mélange des biurets N-sulfénylés de formule I des revendications 1 et 3 avec des agents d'allongement et/ou des produits tensioactifs.